(19) **Europäisches Patentamt**

**European Patent Office**

**Office européen des brevets**

(11) **EP 1 536 747 B1**

(12) **EUROPEAN PATENT SPECIFICATION**

(45) Date of publication and mention
of the grant of the patent:
**21.06.2006 Bulletin 2006/25**

(51) Int Cl.:
***A61F 13/15*** (2006.01)

(21) Application number: **03754512.6**

(86) International application number:
**PCT/US2003/028613**

(22) Date of filing: **11.09.2003**

(87) International publication number:
**WO 2004/024044 (25.03.2004 Gazette 2004/13)**

(54) **ABSORBENT ARTICLE INCLUDING AIRLAID MIXTURE MATERIAL CONTAINING THERMOPLASTIC FIBERS TREATED WITH PHOSPHATE ESTER OR SULFATE ESTER**

ABSORBIERENDER ARTIKEL MIT LUFTGELEGTEN MATERIAL ENTHALTEND MIT PHOSPHATESTERN ODER SULFATESTERN BEHANDELTE THERMOPLASTISCHE FASERN

ARTICLE ABSORBANT COMPORTANT UN MATERIAU DE MELANGE AIRLAID CONTENANT DES FIBRES THERMOPLASTIQUES TRAITEES AVEC UN ESTER DE PHOSPHATE OU UN ESTER DE SULFATE

(84) Designated Contracting States:
**AT BE BG CH CY CZ DE DK EE ES FI FR GB GR HU IE IT LI LU MC NL PT RO SE SI SK TR**

(30) Priority: **12.09.2002 US 410095 P**

(43) Date of publication of application:
**08.06.2005 Bulletin 2005/23**

(73) Proprietor: **THE PROCTER & GAMBLE COMPANY Cincinnati, Ohio 45202 (US)**

(72) Inventors:
• **MINOGUCHI, Ryo Cincinnati, OH 45242 (US)**

• **GRAY, Brian, Francis Cincinnati, OH 45215 (US)**
• **SEKI, Shinichiro Bunkyo-ku, Tokyo 113-0023 (JP)**
• **VENTURA, Peter, David Loveland, OH 45140 (US)**

(74) Representative: **L'Huillier, Florent Charles et al Procter & Gamble Service GmbH Sulzbacher Strasse 40-50 65824 Schwalbach am Taunus (DE)**

(56) References cited:
**EP-A- 0 343 940        US-A- 5 899 893
US-A- 6 120 783**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

FIELD OF THE INVENTION

The present invention relates to absorbent articles. More particularly, the present invention relates to an absorbent article including an airlaid material containing thermoplastic fibers having a surface treated with a surfactant including a phosphate ester, a sulfate ester, or a derivative thereof.

BACKGROUND OF THE INVENTION

Absorbent articles such as sanitary napkins, pantiliners and incontinent pads are devices that are typically worn in the crotch region of an undergarment. More specifically, sanitary napkins, for example, are worn by women in a pair of panties that is normally positioned between the wearer's legs, adjacent to the perineum area. Sanitary napkins are designed to absorb and retain body fluids or discharges (e.g., menses) from the body of women and to prevent body and clothing from soiling.

Leakage protection (i.e., preventing fluids from leaking from a sanitary napkin onto an undergarment) is a primary function of sanitary napkins. It is believed that leakage is caused by combined mechanisms which are: a) slower fluid acquisition from the surface into the inside of the napkin than the discharge rate of menses from the body, and b) poor conformability of the napkin to the body, in particular around creases of the body existing from the exit of the fluids to the anus.

In most cases, this leakage is more acute for thinner sanitary napkins (which typically include an airlaid material composed of cellulosic fibers and bicomponent fibers in the absorbent core) than thicker sanitary napkins (which typically include an agglomeration of cellulosic fibers or "airfelt" in the absorbent core). One reason for this is because their relatively low fluid permeability, in particular with viscous body fluids like menses. It is believed that such low fluid permeability with viscous body fluids is caused by high density of the airlaid material. For example, one airlaid material typically has density of more than 0.08 g/cc as disclosed in USP. No. 5,607,414, issued to Richards et al. on March 4, 1997; and USP. No. 5,916,670, issued to Tan et al. on June 29, 1999. Such high density is required to impart sufficient capillary pressure to the airlaid material. Another reason is high stiffness of the airlaid material which is also caused by high density of the airlaid material. High stiffness of the airlaid material tends to disrupt conformability of the napkin to the body, thus resulting in leakage.

Hence, there is a need for an absorbent article that can effectively prevent leakage with viscous body fluids like menses.

SUMMARY OF THE INVENTION

The invention is directed to an absorbent article, comprising: a liquid pervious topsheet; a backsheet (preferably liquid impervious): an airlaid material disposed between the topsheet and the backsheet.

In one aspect of the invention, the airlaid material includes a) cellulosic fibers, and b) thermoplastic fibers having a surface treated with a surfactant including a phosphate ester, a sulfate ester, or a derivative thereof. The cellulosic fibers and the thermoplastic fibers are bonded together to form a thermally bonded airlaid matrix. In a preferred embodiment, the thermally bonded airlaid matrix has dry density of from about 0.04 to about 0.11 g/cc under pressure of 20 gf/cm$^2$.

It should be noted that the thermally bonded airlaid matrix of the invention is preferably used in or as an absorbent core and/or a fluid acquisition layer which are disposed between the topsheet and the backsheet.

The thermally bonded airlaid matrix of the invention can provide superior fluid handling with viscous body fluids like menses. Such superior fluid handling includes acquisition of the initially discharged fluids, transportation and distribution of the fluids to other remote regions of the airlaid matrix, and containment of the fluids in the airlaid matrix. This superiority is brought by hydrophilic nature of the component fibers (i.e.. both the cellulosic fibers and the thermoplastic fibers) in the thermally bonded airlaid matrix. In particular, by creating the surface of the thermoplastic fibers with the surfactant including a phosphate ester, a sulfate ester, or a derivative thereof, fluid handling with viscous body fluids is much improved. In addition, since the thermally bonded airlaid matrix provides low stiffness due to its low dry density, it can provide improved conformability of the absorbent article to the wearer's body, thereby preventing leakage of body fluids.

The foregoing answers the need for an absorbent article that can effectively prevent leakage with viscous body fluids like menses.

These and other features, aspects, and advantages of the present invention will become evident to those skilled in the art from reading of the present disclosure.

BRIEF DESCRIPTION OF THE DRAWINGS

While the specification concludes with claims particularly pointing out and distinctly claiming the subject matter which is regarded as forming the present invention, it is believed that the invention will be better understood from the following description which is taken in conjunction with the accompanying drawings in which:

Fig. 1 is a perspective view of a sanitary napkin which is one preferred embodiment of the present invention;
Fig. 2 is a cross-sectional view of the sanitary napkin shown in Fig. 1, taken along the line 2-2;
Fig. 3 is a perspective view of a sanitary napkin which is another preferred embodiment of the present invention;
Fig. 4 is a cross-sectional view of the sanitary napkin shown in Fig. 3, taken along the line 4-4; and
Fig. 5 is a graph showing the wicking and absorption properties of thermally bonded airlaid matrices.

DETAILED DESCRIPTION OF THE INVENTION

Herein, "comprise", "include" and "contain" mean that other elements and/or other steps which do not affect the end result can be added. Each of these terms encompasses the terms "consisting of" and "consisting essentially of".
Herein, "absorbent article" refers to articles which absorb and contain body exudates or discharges, and is intended to include sanitary napkins, pantiliners, diapers, and incontinence pads (and other articles worn in the crotch region of a garment).
Herein, "disposable" refers to articles which are intended to be discarded after a single use, composted, or otherwise disposed of in an environmentally compatible manner. (That is, they are not intended to be laundered or otherwise restored or reused as an absorbent article.)
Herein, "sanitary napkin" refers to articles which are worn by women adjacent to the pudendal region which are intended to absorb and contain discharges from the body (e.g., blood, menses, and urine).
Herein, "joined" encompasses configurations in which an element is directly secured to another element by affixing the element directly to the other element; configurations in which the element is indirectly secured to the other element by affixing the element to intermediate member(s) which in turn are affixed to the other element; and configurations in which one element is integral with another element, i.e., one element is essentially part of the other element.
All percentages, ratios and proportions used herein are by weight unless otherwise specified. A. Thermally Bonded Airlaid Matrix (hereinafter referred to as "TBAM")

1. Cellulosic Fibers

Cellulosic fibers useful in the present invention include naturally occurring, unmodified cellulosic fibers, as well as modified cellulosic fibers. Examples of suitable unmodified/modified cellulosic fibers include wood pulp, chemically modified wood pulp, rayon, cotton, cotton linter, kenaf pulp, chitin fibers, chitosan fibers, Esparto grass, bagasse, kemp, flax, jute, and the like.
The cellulosic fibers used in the present invention are hydrophilic mainly because of a number of hydroxyl groups in cellulosic molecules. Herein, "hydrophilic" describes fibers, or surfaces of fibers, that are wettable by aqueous fluids (e.g., body fluids) deposited on these fibers.
For reasons of availability and cost, wood pulp fibers are the most preferred cellulosic fibers for use in the present invention. Suitable wood pulp fibers can be obtained from known chemical processes such as the Kraft and sulfite processes. It is especially preferred to derive these wood pulp fibers from southern soft woods due to their premium absorbency characteristics. One example of preferred Kraft pulp fibers is available from Rayonier Inc., GA, USA, under the trade name Rayfloc J-LD-E.
The cellulosic fibers useful in the present invention may include chemically stiffened cellulosic fibers. Herein, "chemically stiffened cellulosic fibers" means cellulosic fibers that have been stiffened by chemical means to increase the stiffness of the fibers under both dry and wet conditions. Such means can include addition of a chemical stiffening agent that, for example, coats and/or impregnates the fibers. Such means can also include stiffening of the fibers by altering the chemical structure, for example, by crosslinking polymer chains. In one preferred embodiment, the cellulosic fibers are coated or impregnated with a chemical stiffening agent. Preferred agents for such a chemical stiffening agent include polycarboxylates such as a citric acid. The polycarboxylate stiffening agents and a process for making stiffened fibers are described in USP. No. 5,190,563, issued to Herron et al. on March 2, 1993.

2. Thermoplastic Fibers

Thermoplastic fibers have an ability to form numerous interfiber bond sites within the TBAM. Suitable thermoplastic materials for the thermoplastic fibers can be made from any thermoplastic polymer that can be melted at temperatures that will not extensively damage the cellulosic fibers. Preferably, the melting point of this thermoplastic material will be less than about 190 °C, and preferably between about 70 °C and about 170 °C.

The thermoplastic materials of the thermoplastic fibers can be made from a variety of thermoplastic polymers, including polyolefins such as polyethylene and polypropylene, polyesters, copolyesters, polyvinyl acetate, polyethylvinyl acetate, polyvinyl chloride, polyvinylidene chloride, polyacrylics, polyamides, copolyamides, polystyrenes, polyurethanes and copolymers of any of the foregoing.

Suitable thermoplastic fibers can be made from a single polymer (monocomponent fibers) or more than one polymer (e.g., bi-, tri- or multi-component fibers). Bicomponent fibers are the most preferred.

Bicomponent fibers are generally defined as fibers made of two thermoplastic polymers. Typically they include a portion whose melting point is less than about 170 °C, and preferably between about 70 °C and about 170 °C so that it can be melted with thermal treatment and solidified with subsequent cooling, and another portion whose melting point is more than about 170 °C so that it cannot be melted and maintain its shape and strength during the thermal treatment. Thus, the bicomponent fibers function as binders. Preferred bicomponent fibers have a sheath/core structure.

Suitable bicomponent fibers have the following polymer combinations: polyethylene/polypropylene, polyethylene/polyester, polyethylvinyl acetate/polypropylene, copolyester/polyester, and the like. Particularly preferred bicomponent fibers are those having a sheath of polyethylene, polyethylvinyl acetate or a lower melting copolyester, and a core of polypropylene or polyester. Examples of preferred bicomponent fibers are available, for example, from Chisso Corporation, Osaka, Japan, under the trade names CHISSO ES and CHISSO ESC; and from FiberVisions A/S, Varde, Denmark, under Code Nos. T-198 and T-255. In a preferred embodiment, the bicomponent fibers include a modified polyolefin (e.g., a modified polyethylene in the sheath) to form a hydrogen bond or a covalent bond between the bicomponent fibers and the cellulosic fibers so that it can impart an improved adhesion between the bicomponent fibers and the cellulosic fibers.

Such bicomponent fibers are disclosed in USP. No. 5,981,410 issued to Hansen et al. on November 9, 1999, wherein bicomponent fibers having polyethylene in the sheath grafted with an unsaturated dicarboxylic acid or an anhydride thereof, e.g., with maleic acid or maleic anhydride, are disclosed, and in Japan Patent No. 2001329432 issued to Miyauchi et al. on November 27, 2001, wherein bicomponent fibers having acid modified polyethylene in the sheath are disclosed, and available, for example, from Chisso Corporation, Osaka, Japan, under the trade name CHISSO INTAK.

The percentage of the bicomponent fibers needs to be high enough to impart adequate structural stability and strength to the airlaid matrices, and low enough to maintain capillary structure from the cellulosic fibers. It is preferably from about 10% to about 30% and more preferably from about 15% to about 25%.

The length of the thermoplastic fibers (or the bicomponent fibers) can vary depending upon the properties of the thermoplastic fibers, and the desired characteristics of the resultant thermally bonded matrix. Preferred bicomponent fibers have a length from about 1.5 mm to about 10 mm, more preferably from about 2.0 mm to about 6.0 mm, and yet more preferably from about 3.0 mm to about 5.0 mm. The diameter (caliper) of the thermoplastic fibers can also vary depending upon the desired characteristics of the resultant thermally bonded matrix. The diameter of these fibers is typically defined in terms of either denier (i.e., grams per 9000 meters) or decitex (i.e., grams per 10,000 meters). Suitable bicomponent fibers can have a decitex from about 1.0 to about 10, preferably from about 1.4 to about 5.0, and yet more preferably from about 1.7 to about 2.2.

3. Surfactant and Surface Treatment of Fibers

The thermoplastic fibers of the present invention has a surface treated with an anionic surfactant. This treatment is a hydrophilic treatment of the thermoplastic fibers and is essential to achieve the object of the present invention via high fluid permeability and high capillary pressure simultaneously. Any methods for surface treatment of fibers known in the art can be used for this hydrophilic treatment. A preferred method is disclosed in, for example, USP. No. 5,607,414 issued to Richards et al. on March 4, 1997 wherein the treatment is achieved by coating the surface of fibers with an anionic surfactant. Preferably, the anionic surfactant is selected from the group consisting of phosphate esters, sulfate esters, and their derivatives.

If desired, the surface treatment with the anionic surfactant may be accompanied with other known surfactants, such as nonionic surfactants like ethoxylates, glycerol esters, glycol esters, sorbitan esters, fatty acid esters, and the other anionic surfactants such as fatty acids, fatty acid alkali metal salts, or the mixture thereof.

Preferably, the surface treatment is performed in a liquid form of the anionic surfactant with an appropriate solvent. In addition to water, various alcohols can be used as the solvent. In the preferred embodiment wherein the surfactant includes a phosphate ester or a sulfate ester, ethylene glycol, propylene glycol or glycerin can be preferably used as a solvent.

The anionic surfactant solution can be applied onto the thermoplastic fibers by any of various techniques and apparatus used for applying solutions to materials including coating, dumping, pouring, dropping, spraying, atomizing, condensing, or immersing the anionic surfactant onto the thermoplastic fibers. As used herein, the term "applied onto" means that at least a portion of the surface of at least one of the thermoplastic fibers has the anionic surfactant on it. Thus, the anionic surfactant may be applied onto only some of the thermoplastic fibers, onto all of the thermoplastic fibers, onto only a portion of the surface of some or all of the thermoplastic fibers, or onto the entire surface of some or all of the thermoplastic fibers. Preferably, the anionic surfactant is coated onto the entire surface of most, preferably all, of the thermoplastic fibers so as to enhance the hydrophilicity of the thermoplastic fibers.

Alternatively, the treatment can be carried out by incorporating the anionic surfactant into the polymeric resins of the thermoplastic fibers during their production so that the surface of the thermoplastic fibers becomes treated with the anionic surfactant.

In a more preferred embodiment, the thermoplastic fibers have a surface treated with a surfactant including a phosphate ester, a sulfate ester, or a derivative thereof. Such a surfactant is particularly preferred since it can provide a drastic effect on the hydrophilicity and/or wettability of the surface of the thermoplastic fibers particularly with viscous body fluids like menses, which can be attributed to very low contact angle i.e. less than 20 degree with menses (or "Test Fluid" described hereafter). Such very high wettability and/or low contact angle is believed due to the high affinity of such a surfactant with hematological and mucous components of such body fluids.

Preferred phosphate esters and sulfate esters are disclosed in, for example, USP. No. 3,926,816 issued to Cohen et al. on Dec. 16, 1975; USP. No. 5,614,574 issued to Sheth on Mar. 25, 1997; and Japan Patent No. 2001159078 issued to Iwata et al. on June 12, 2001.

A preferred phosphate ester or its derivative is selected from the group consisting of alkyl phosphate esters, alkyl phenol phosphate esters, and their derivatives such as ethoxylated forms. Preferably, the phosphate ester or its derivative including a linear alkyl phosphate ester having a hydrocarbon chain length of from $C_6$ to $C_{22}$ in the form of an alkali salt, and more preferably from $C_6$ to $C_{16}$ in the form of an alkali salt such as potassium or sodium salts.

A preferred sulfate ester or its derivative is selected from the group consisting of alkyl sulfate esters, alkyl phenol sulfate esters, and their derivatives such as ethoxylated forms. Preferably, the sulfate ester or its derivative including a linear alkyl sulfate ester having a hydrocarbon chain length of from $C_6$ to $C_{22}$ in the form of an alkali salt, and more preferably from $C_6$ to $C_{16}$ in the form of an alkali salt such as potassium or sodium salts.

In one preferred embodiment, the surfactant includes a mixture of n-dodecyl ($C_{12}$) phosphate ester potassium salts and n-octyl ($C_8$) phosphate ester potassium salts.

The percentage of the surfactant for the thermoplastic fibers can vary depending upon the type of the surfactant and expected degree of hydrophilicity or wettability. Typically, it is from about 0.01% to about 10%, preferably from about 0.1 % to about 5%.

One preferred example of the thermoplastic fibers having a surface treated with a surfactant including a phosphate ester is available from Chisso Corporation, Osaka, Japan, under Code No. XESC1015, which is acid modified polyethylene (in the sheath) / polypropylene (in the core) thermoplastic fibers (1.7 decitex, 5.0 mm length) coated with a mixture of n-dodecyl ($C_{12}$) phosphate ester potassium salts and n-octyl ($C_8$) phosphate ester potassium salts.

4. Absorbent Gelling Material (AGM)

The TBAM of the present invention preferably includes an absorbent gelling material (hereinafter referred to as "AGM") when it is used in an absorbent core. Such an AGM forms a hydrogel upon contact with water. Any AGMs known in the art (including those referred to as hydrogels, superabsorbent, or hydrocolloid materials) can be used in the TBAM of the present invention.

In a preferred embodiment where the thermoplastic fibers have a surface treated with an anionic surfactant, the TBAM of the present invention further includes from about 5% to about 50%, preferably from about 10% to about 30% of an AGM. Due to low dry density of the TBAM, it can provide enough space(s) in it for AGM to swell upon fluid absorption, and enable an expected absorption capacity. In addition, low dry density of the TBAM can help keep low resiliency in both dry and wet conditions. Thus, low dry density of the TBAM provides improved conformability as well as absorption capacity which are expected as an absorbent core.

In preferred embodiments in particular for catamenial absorbent articles such as sanitary napkins, hemophilic AGMs are preferably used. Herein, "hemophilic AGM" is referred to AGMs which have an absorption capacity of more than about 7g/g of a sheep blood base test fluid (hereinafter referred to as "Test Fluid"), which exhibits viscosity of 120+20 cP at 9.0 1/s of shear rate, equivalent to the highest viscosity of real menses. Test Fluid contains defibrinated sheep blood and gastric mucin. A method for making Test Fluid is described in the "Test Methods" section.

Preferred hemophilic AGMs are disclosed in USP. No. 5,807,361 issued to Kajikawa et al. on September 15, 1998. A preferred hemophilic AGM is available from Nippon Shokubai Co., Ltd., Osaka, Japan, under Code Nos. PX3-L-1083, and PX3-L-1091.

5. Other Optional Components

The TBAM of the present invention may optionally include other components. For example, the TBAM can includes some synthetic fibers that do not function as binders like the thermoplastic fibers but can change fluid transportation characteristics of the TBAM to some extent. Such synthetic fibers include polyester fibers having a highly dimensional cross-section (available from, e.g., Toyobo Co. Ltd., Osaka, Japan, under the trade name TRIACTOR), hollowed polyester fibers (available from, e.g., Teijin Ltd., Osaka, Japan, under the trade name WELKY), and hydrophilic nylon fibers (available from, e.g., AlliedSignal Inc., VA, USA under the trade name HYDROFIL). If desired, the TBAM can also include some polymeric particles such as polyethylene powders to alter mechanical properties of the airlaid materials to some extent. The TBAM can further include inorganic particles such as silica, silicates, zeolite, almina, and the like to impart any additional characteristics such as dissociating or trapping odors.

6. Structure of TBAM

By using an airlaid process, the cellulosic fibers and the thermoplastic fibers (and other optional components described above) are mixed to form an airlaid mixture material. In one preferred embodiment, the cellulosic fibers and the thermoplastic fibers are mixed uniformly within the entire airlaid mixture material. However, in an alternative preferred embodiment, the TBAM can have heterogeneous distribution of component materials particularly across the thickness so as to impart different desired properties to the surfaces and the center portion sandwiched with the surfaces, respectively. For example, if particulate AGM is included, the AGM particles are incorporated only in the center portion of the airlaid materials for ensuring containment of the AGM, while the surfaces only include the cellulosic fibers and the thermoplastic fibers. Further, the surface(s) can include more thermoplastic fibers than the center portion of the airlaid materials so that dusting from the airlaid materials can be reduced. Such heterogeneous compositions can be prepared by depositing discrete component materials from a plurality of distributor units sequentially in the air-laying system.

The TBAM is formed by bonding the airlaid mixture of the cellulosic fibers and the thermoplastic fibers (and other optional components described above) together such that it has dry density of from about 0.04 g/cc to about 0.11 g/cc, preferably from about 0.05 g/cc to about 0.10 g/cc, more preferably from about 0.05 g/cc to about 0.08 g/cc, under pressure of 20 gf/cm$^2$.

B. Process for TBAM

The TBAM of the present invention is produced by airlaying a mixture of the cellulosic fibers and the hydrophilic thermoplastic fibers (and other optional components) described above in an air flow and subsequently giving thermal treatment to the airlaid mixture. In general, this airlaying can be carried out by metering an airflow containing a mixture of the cellulosic fibers and the thermoplastic fibers (and other optional components described above), in substantially dry condition, onto a typically horizontally moving wire forming screen. Any airlaying systems know in the art can be used. Suitable systems and apparatus for this airlaying are disclosed, for example, in USP. No. 4,144,619 issued to White et al. on March 20, 1979; USP. No. 4,157,724 issued to Persson on June 12, 1979, and reissued December 25, 1984 as Re. 31,775; and USP. No. 4,494,278 issued to Kroyer et al. on January 22, 1985.

Particularly desirable systems for the airlaying are available from Dan-webforming International A/S, Risskov, Denmark, and M&J Fibretech A/S, Horsens, Denmark.

The TBAM of the present invention is produced with thermal treatment following air-laying, so as to activate the thermoplastic fibers comprised and form the bond sites between such thermoplastic fibers and/or between such thermoplastic fibers and the cellulosic fibers. This thermal treatment can be achieved by subjecting the airlaid web into the oven where heated air is circulated with appropriate control of temperature and flow volume. Typically, this oven can be attached to the airlaying system mentioned above and the airlaid web from airlaying system can be guided into the oven continuously. Temperature of the oven must be high enough for a portion of the thermoplastic fibers to be melted and form the bond sites and low enough for another portion of the thermoplastic fibers not to be melted and maintain its shape and strength. It is preferably from about 120 °C to about 170 °C, and more preferably from about 130 °C to about 160 °C.

Upon melting, at least a portion of the melted thermoplastic material migrates to the intersections of the fibers, typically due to interfiber capillary gradients. These intersections become bond sites for the component fibers. When cooled, the thermoplastic material at these intersections solidify to form the bond sites that hold the web or matrix of fibers. Amongst its various effects, bonding at these fiber intersections increases resilience and strength for maintaining low density structure of TBAM.

The air-laying system preferably includes calendaring or compaction rolls to control thickness and density of the resulting TBAM, which is from about 0.04 g/cc to about 0.11 g/cc at 20 gf/cm$^2$. Such calendaring or compaction rolls can be provided at a stage of the air-laying system before the thermal treatment and/or after the distributor unit (particularly in the case where a plurality of distributor units are used). In a preferred embodiment, a carrier tissue is placed over the forming screen to prevent the components applied from the distributor units onto the forming screen from passing through the forming screen. However, if desired this carrier tissue can be removed when it is incorporated into absorbent articles.

C. Absorbent Article

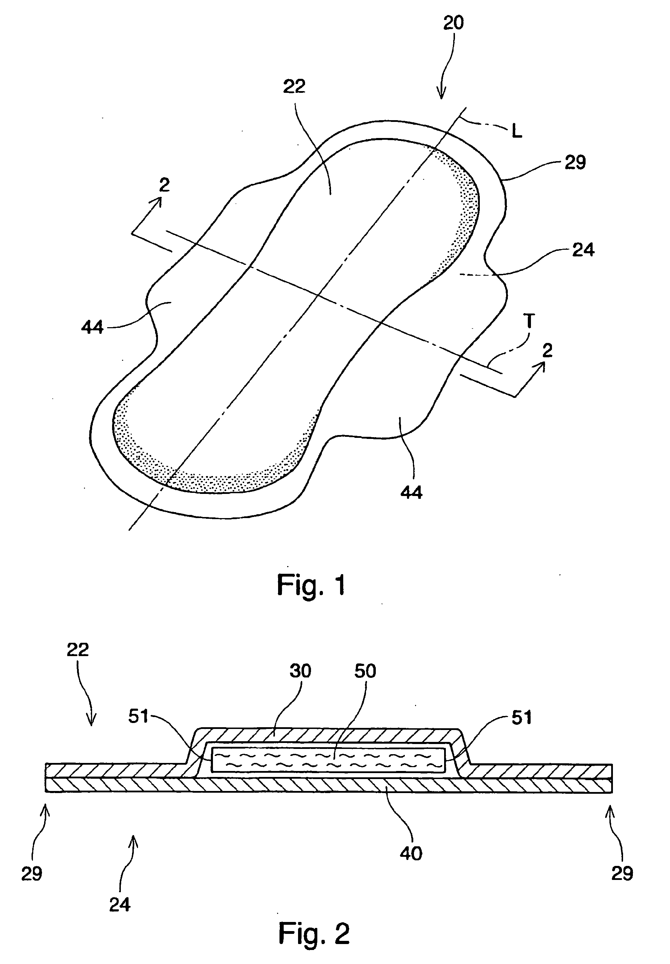

Fig. 1 is a perspective view of a sanitary napkin 20 (i.e., a disposable absorbent article) which is one preferred embodiment of the present invention. Referring to Fig. 1, the sanitary napkin 20 and its component members have a body facing surface 22, and a garment facing surface 24 opposed to the body facing surface 22. The sanitary napkin 20 shown in Fig. 1 is viewed from the body facing surface 22. The sanitary napkin 20 has a circumferential edge 29 which defines the outermost edge of the sanitary napkin 20.

The sanitary napkin 20 has two centerlines, a longitudinal centerline L and a transverse centerline T. Herein, "longitudinal" refers to a line, axis or direction in the plane of the sanitary napkin 20 that is generally aligned with (e.g., approximately parallel to) a vertical plane which bisects a standing wearer into left and right body halves when the sanitary napkin 20 is worn. Herein, "transverse" or "lateral", are interchangeable, and refer to a line, axis or direction which lies within the plane of the sanitary napkin 20 that is generally perpendicular to the longitudinal direction.

Fig. 2 is a cross-sectional view of the sanitary napkin 20 shown in Fig. 1, taken along the line 2-2. The sanitary napkin 20 includes three primary components. These include a liquid pervious topsheet 30, a liquid impervious backsheet 40, and an absorbent core 50 disposed between the topsheet 30 and the backsheet 40. The top surface of the topsheet 30 forms the body facing surface 22 of the sanitary napkin 20, while the bottom surface of the backsheet 40 forms the garment facing surface 24 of the sanitary napkin 20.

The absorbent core 50 is capable of receiving, absorbing or retaining body fluids discharged. The absorbent core 50 is preferably compressible and conformable. The absorbent core 50 can include a single layer (or a plurality of layers) of the TBAM of the present invention. In the preferred embodiment shown in Figs. 1 and 2, the absorbent core 50 includes one layer of the TBAM of the present invention.

The absorbent core 50 can have a wide variety of sizes and shapes. The core edge 51 of the absorbent core 50 thus can take any shape in its top plan view. Preferred shapes for the core edge 51 include an oval, a rectangle, a dog-bone (as shown in Fig. 1), an hourglass, and a combination thereof.

The topsheet 30 and the backsheet 40 extend outward from the core edge 51. The topsheet 30 and the backsheet 40 preferably extend in both the longitudinal and transverse directions to reach the circumferential edge 29 of the sanitary napkin 20. They are joined together by any means known in the art. Preferably, these portions of the topsheet 30 and the backsheet 40 are joined using adhesives over substantially the entire portions that extend beyond the core edge 52 of the absorbent core 50. In a preferred embodiment, the topsheet 30 and backsheet 40 are densified by application of pressure or heat and pressure to form a crimp seal.

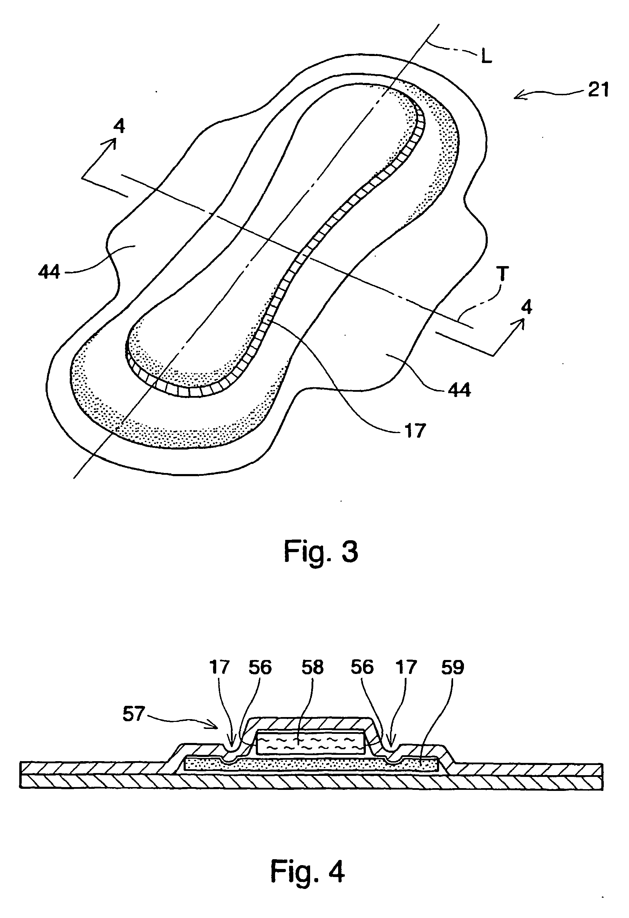

Fig. 3 is a perspective view of a sanitary napkin 21 which is another preferred embodiment of the present invention. Referring to Fig. 3, the sanitary napkin 21 basically has a similar structure to that of the sanitary napkin 20 shown in Fig. 1 except the structure of an absorbent core 57. Specifically, compared with the sanitary napkin 20 shown in Figs. 1 and 2, the absorbent core 57 of the sanitary napkin 21 include two layers, at least one of them includes the TBAM of the present invention, and have similar dog-bone shapes with different sizes.

Fig. 4 is a cross-sectional view of the sanitary napkin 21 shown in Fig. 3, taken along the line 4-4. Referring to Fig. 4, the absorbent core 57 includes an upper layer (or a first layer) 58 having a circumferential edge 56 and a lower layer (or a second layer) 59. At least one of the upper and lower layers 58 and 59 includes the TBAM of the present invention. The both of the upper and lower layers 58 and 59 can include the same TBAM of the present invention. However, the upper and lower layers 58 and 59 can include different compositions, percentages of components, sizes, shapes, basis weight, and/or density of the TBAM of the present invention.

In a preferred embodiment, the upper layer 58 has lower density and lower percentage of AGM than the lower layer 59. This design can help the upper layer 58 to drain viscous body fluids like menses to the lower layer 59 smoothly, without causing "gel blocking". Herein, "gel blocking" refers to porosity reduction in an absorbent core caused by swollen AGM when it absorbs fluids and becomes hydrogels. Preferably, the upper and lower layers 58 and 59 have density of from about 0.04 g/cc to about 0.08 g/cc and from about 0.07 g/cc to about 0.11 g/cc at 20gf/cm$^2$, respectively. The upper and lower layers 58 and 59 preferably have percentage of the AGM from about 0% to about 20% and from about 15% to about 30%, respectively.

The upper and lower layers 58 and 59 can have either same or different sizes and/or shapes. In a preferred embodiment, the lower layer 59 is greater in both the longitudinal and transverse directions, as shown in Figs. 3 and 4.

In the embodiment shown in Figs. 3 and 4, the upper layer 58 has a smaller area and greater thickness than the lower layer 59 so that the sanitary napkin 21 shows a clear profiling, which can offer an improved conformability of the napkin 21 to the body shape thereby providing a better leakage protection and wearing comfort. Preferably, the upper airlaid layer 58 has thickness of from about 1 mm to about 5 mm, and the lower airlaid layer 59 has thickness of from about 0.5 mm to about 2 mm. Preferably, the upper layer 58 has a dog-bone shape (as shown in Fig. 3) so that the body facing surface 22 can fit to the creases at the prudendal region of female body.

As shown in Figs. 3 and 4, the body facing surface 22 of the napkin 21 is preferably embossed on the lower layer 59 along (preferably slightly outside) the edge 56 of the upper layer 58 to form an embossed channel 17. The embossed channel 17 can be formed by any methods known in the art. In a preferred embodiment, the embossed channel 17 is formed by a hot press at about 70°C and 6.7 kgf/cm$^2$, from the topsheet 30.

This embossed channel 17 can provides users with a visual impression of concentrated capacity at the center region of the napkin 21, while ensuring the integrity of the upper layer 58. Such an embossed channel 17 can be formed by applying a pressure with heat to the body facing surface 22 of the topsheet 2. If desired, an additional embossed channel(s) can also be provided on the upper layer 58 for easy bending or buckling (not shown in Figs.). The pattern of such an additional embossed channel(s) typically include a continuous line(s) which can be straight and/or curved. The embossed channels can have any pattern known in the art.

In the embodiment where the other of the upper and lower layers 58 and 59 includes (or is formed by) a different material from the TBAM of the present invention, that layer can include any of a wide variety of liquid-absorbent materials commonly used in absorbent articles, such as comminuted wood pulp, which is generally referred to as airfelt. Examples of other suitable absorbent materials for that layer include creped cellulose wadding; meltblown polymers including coform; chemically stiffened, modified or cross-linked cellulosic fibers; synthetic fibers such as crimped polyester fibers; peat moss; tissue including tissue wraps and tissue laminates; absorbent foams; absorbent sponges; AGMs; multi-bonded airlaid nonwoven material; and any combinations or mixtures thereof.

The topsheet 30 is preferably compliant, soft feeling, and non-irritating to the wearer's skin. The topsheet 30 is liquid pervious or permeable, permitting body fluids (e.g., menses and/or urine) to readily penetrate through its thickness. Preferred liquid pervious materials may be manufactured from a wide range of materials such as woven and nonwoven materials (e.g., a nonwoven web of fibers); polymeric materials such as apertured formed thermoplastic films, apertured plastic films, and hydroformed thermoplastic films; porous foams; reticulated foams; reticulated thermoplastic films; and thermoplastic scrims.

A particularly suitable topsheet material for use in the sanitary napkins includes an apertured formed film. Apertured formed films are preferred for the topsheet 30 because they are pervious to body fluids and, if properly apertured, have a reduced tendency to allow liquids to pass back through and rewet the wearer's skin. Thus, the body facing surface 22 of the formed film remains dry, thereby reducing body soiling and creating a more comfortable feel for the wearer.

Preferably, the body facing surface 22 of the topsheet 30 is hydrophilic so that liquids will be transferred through the topsheet 30 more readily. A preferred material for the topsheet 30 is a macroscopically expanded, three-dimensional formed polyethylene film. One preferred apertured formed film material for the topsheet 30 is available from Tredegar Film Products, IN, USA under Code No. X-15507.

The backsheet 40 is impervious to body fluids and is preferably manufactured from a thin plastic film, although other flexible liquid impervious materials may also be used. Herein, "flexible" refers to materials which are compliant and will readily conform to the general shape and contours of the human body. The backsheet 40 prevents the body fluids absorbed and contained in the absorbent core 50 from wetting articles which contact the absorbent article such as pants, pajamas and undergarments. The backsheet 40 may thus include a woven or nonwoven material, polymeric films such as thermoplastic films of polyethylene or polypropylene, or composite materials such as a film-coated nonwoven material. The backsheet 40 can be embossed and/or matte finished to provide a more clothlike appearance.

The backsheet 40 can include a single layer material, or two or more layers of materials. The backsheet 40 preferably has thickness of from about 0.012 mm (0.5 mil) to about 0.051 mm (2.0 mils). In a preferred embodiment, the backsheet 40 is a single layer polyethylene film. A preferred polyethylene film is available from Clopay Corporation, OH, USA under Code No. P18-1401.

The backsheet 40 preferably has a microporous structure which can permit vapors to escape from the absorbent core (often called "breathable backsheet") while still preventing body fluids from passing through the backsheet 40. A preferred microporous polyethylene film is available from Mitsubishi Chemical Corporation, Tokyo, Japan, under the trade name NAP.

The sanitary napkin 20 can include an optional pair of flaps (or wings) 44 which are formed by the transversely extended portion of the topsheet 30 and the backsheet 40 from the absorbent core 50 as shown in Fig. 2. Suitable flaps are described in USP. No. 5,389,094 issued to Lavash, et al. on February 14, 1995; and USP. No. 5,558,663 issued to Weinberger, et al. on September 24, 1996.

In a preferred embodiment, a fluid acquisition layer (or a secondary topsheet) is additionally disposed between the topsheet 30 and the absorbent core 50 or 57 (not shown in Figs.). The fluid acquisition layer helps the transportation of discharged body fluids received by the topsheet 30 to other parts of the acquisition layer and the absorbent core 50. A preferred material for the fluid acquisition layer is a hydrophilic carded nonwoven available from Rengo Nonwoven Products Co. Ltd., Okayama, Japan, under Code No. 623609. In an alternative preferred embodiment, the fluid acquisition layer includes a single layer (or two or more layers if desired) of the TBAM of the present invention.

The sanitary napkins 20 and 21 can generally have any thickness including relatively thick, intermediate thickness, relatively thin, or even very thin (or "ultra thin"). Preferred "ultra-thin" sanitary napkins which preferably have a caliper of less than about 3 mm are described in USP. Nos. 4,950,264 and 5,009,653 issued to Osborn. The embodiments of the sanitary napkins 20 and 21 shown in Figs. 1-4 are examples of an ultra-thin sanitary napkin. The sanitary napkins 20 and 21 may also be relatively flexible, so that they are comfortable for the wearer.

Additionally or in an alternative preferred embodiment, the topsheet 30 is joined to the absorbent core 50 (or the secondary topsheet if exists) by a fusion bond (i.e., an application of heat/pressure) known in the art. The pattern of the fusion bond can include a continuous line(s) and/or a number of discrete portion of fusion bonds.

The garment facing surface 24 of the sanitary napkin 20 (or the backsheet 40) is provided with adhesive fasteners for attaching the sanitary napkin 20 to the wearer's undergarment. The garment facing surface of the flaps 44, adjacent the distal edges of the flaps 44, is also provided with a flap adhesive fastener.

D. Test Methods

1. Preparation of Sheep Blood Base Test Fluid

Many fluids are known for representing menses in laboratory testing. The composition we have selected represents the highest viscosity of menses and is prepared as below.

1) Prepare 1000 mL solution of 1.38 ±0.005 g of sodium phosphate monobasic monohydrate and 8.50 ±0.005g of sodium chloride with distilled water (Solution A).

2) Prepare 1000 mL solution of 1.42 ±0.005 g of sodium phosphate dibasic anhydrous and 8.50 ±0.005 g of sodium chloride with distilled water (Solution B).

3) Add Solution A to 450 ±10 mL of Solution B while slowly mixing until pH of the solution reaches to 7.2 ±0.1 (Solution C).

4) Dissolve about 120g of gastric mucin powder, which is available, for example, from American Laboratories, Inc., NE, USA, in 460 ±10 mL of Solution C stirring with any propeller electric stirrer, which is available, for example, from IKA Works, Inc., NC. USA, under the trade name Model RW 16 Basic, at stirring speed with no splash for 2.5 hours at 70 °C (Solution D).

5) After cooling down below 40°C, Add 1.8 ±0.2 mL of 10% v/v lactic acid solution which is available, for example, from VWR International, PA, USA, to Solution D and stir with any propeller electric stirrer mentioned above for 2 minutes (Solution E).

6) Autoclave Solution E at 121 °C for 15 minutes and take the solution out of the autoclave after being cooled down below 60 °C.

7) Mix 500 mL of Solution E and 500 mL of fresh, sterile defibrinated sheep blood available, for example, from Cleveland Scientific Ltd., OH, USA. (hereinafter referred to as "Test Fluid").

8) Measure viscosity of Test Fluid at 23 ±1 °C and shear rate of 9.0 1/s using with any viscometer which can define shear rate precisely, for example, a programmable viscometer available from Brookfield Engineering Laboratories, Inc., MA, USA under the trade name LV DV-II+ (CP-40).

9) Confirm the viscosity of Test Fluid is 120+20 cP. If the viscosity is higher than this range, appropriately reduce gastric mucin powder in the step 4) and repeat the steps 4) - 8) until the viscosity is within the range. If the viscosity is lower than this range, appropriately increase gastric mucin powder in the step 4) and repeat the steps 4) - 8) until the viscosity is within the range.

2. Absorption Test for AGM

1) Evenly spread 1 g of particles or any other forms of AGM in a petri dish having inner diameter of 100 mm.

2) Pour 10 g of Test Fluid prepared above evenly over the AGM in 10 sec.

3) After 5 minutes, put a pile of filter papers available, for example, from Empirical Manufacturing Company, Inc., OH, USA, under Code No. 989 cut into a circle of 100 mm diameter, which are weighed beforehand (W1 [g]), then a weight of 2 kg having outer diameter of 100 mm immediately over the AGM. Filter papers must be many enough for them to absorb excess fluid completely.

4) After 1 minute, remove the weight and the filter papers. Confirm no excess fluid remains.

5) Weigh the filter papers (W2 [g]).

6) Calculate absorption of Test Fluid by the AGM as below;

EP 1 536 747 B1

$$Absorption\ [g/g] = (10\ [g] - (W2\ [g] - W1\ [g])) / 1\ [g]$$

The AGM which meets this definition can absorb and retain menses very effectively in the TBAM.

Table 1 shows preferred AGMs for the TBAM.

Table 1

| AGM | Absorption |
|---|---|
| Nippon Shokubai PX3-L-1091 (hemophilic) | 7.3 g/g |
| Nippon Shokubai Aqualic CA L-74 | 3.4 g/g |
| Nippon Shokubai Aqualic CA L-401 | 5.8 g/g |
| Stockhausen Nalco N1180 | 4.7 g/g |

3. Wicking Test

This test measures capillary pressure of sample materials as the quantity of Test Fluid which left on Saran film, available from S.C. Johnson & Son, Inc., WI, USA. After the film is spread on a flat table, 100 uL of Test Fluid is poured therein. A piece of test sample (5 cm x 5 cm) is put over Test Fluid for 5 sec with no additional weight. Capillary pressure is measured as the quantity of Test Fluid left on the film.

4. Absorption Test

After a piece of test sample (5 cm x 5 cm) is put on a flat table, 0.5 mL of Test Fluid is dropped from a height of 0.5 cm over the sample. The absorption time is measured as the time length from the time when Test Fluid hits the surface of the test sample to the time when it is confirmed that Test Fluid completely disappears on the surface of the test sample by naked eyes (i.e. completion of absorption).

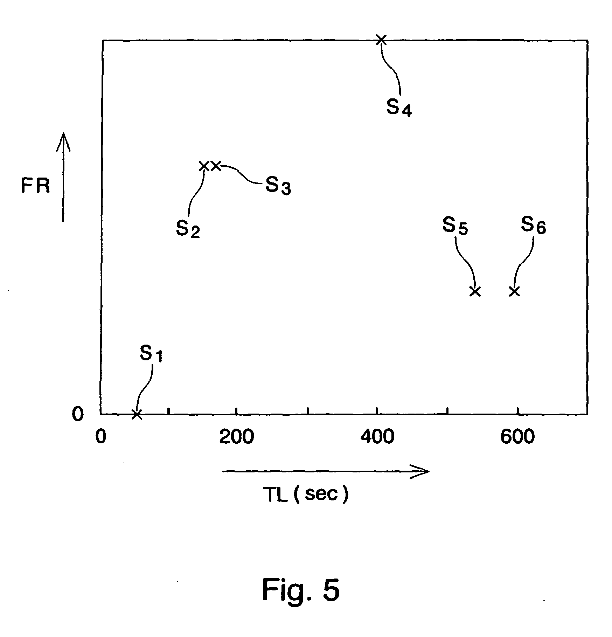

Table 2 and Fig. 5 show a comparison of the TBAMs which are embodiments of the invention (Samples 1 and 5) with the other reference materials (Samples 2-4 and 6). In Fig. 5, the vertical axis shows the quantity of Test Fluid which remains (FR), and the horizontal axis shows the time length required for completion of absorption (TL).

As clearly seen, only Sample 1 (S1) shows high fluid permeability and high wicking (i.e., high capillary pressure), simultaneously. All Samples 1-6 have a basis weight of 150 g/m2 and include 18% of bicomponent fibers. Only Sample 6 includes AGM. Compared to Sample 6, Sample 5 delivers faster absorption maintaining wicking performance even without the benefit of AGM.

Table 2

| Samples (S1-S6) | Absorption Test (Absorption Time) | Wicking Test (Fluid Remain) |
|---|---|---|
| Sample 1<br>Dry Density at 20 gf/cm$^2$: 0.06 g/cc<br>Surface Treatment: Alkyl Phosphate Ester Potassium Salts | 54 sec | 0 |
| Sample 2<br>Dry Density at 20 gf/cm$^2$: 0.06 g/cc<br>Surface Treatment: Fatty Acid Ethoxylates | 150 sec | ++ |
| Sample 3<br>Dry Density at 20 gf/cm$^2$: 0.06 g/cc<br>Surface Treatment: Fatty Acid Sorbitan Esters and Fatty Acid Potassium Salts | 156 sec | ++ |
| Sample 4<br>Dry Density at 20 gf/cm$^2$: 0.06 g/cc<br>Surface Treatment: None | 401 sec | +++ |
| Sample 5 | | |

Table continued

| Samples (S1-S6) | Absorption Test (Absorption Time) | Wicking Test (Fluid Remain) |
|---|---|---|
| Dry Density at 20 gf/cm$^2$: 0.13 g/cc<br>Surface Treatment: Alkyl Phosphate Ester Potassium Salts | 538 sec | + |
| Sample 6<br>Dry Density at 20 gf/cm$^2$: 0.13 g/cc<br>Surface Treatment: None; 17% of AGM | 593 sec | + |
| Note: the number of "+" is proportional to the amount of remaining Test Fluid. | | |

E. Examples of TBAMs

EXAMPLE 1

One layer of TBAM is produced from 82% of Kraft pulp fibers available from Rayonier Inc., GA, USA, under the trade name Rayfloc J-LD-E ("Pulp Fibers") and 18% of acid modified polyethylene (in the sheath) / polypropylene (in the core) bicomponent fibers (1.7 decitex, 5.0 mm length) coated with a mixture of n-dodecyl (C12) phosphate ester potassium salts and n-octyl (C8) phosphate ester potassium salts, available from Chisso Corporation, Osaka, Japan, under Code No. XESC1015 ("Bico Fibers") using an air-laying system available from M&J Fibretech A/S, Horsens, Denmark ("Air-lay System"). The subsequent thermal treatment is carried out at about 142 °C. This material has basis weight of 100 g/m2 and thickness of about 2.0 mm at 20 gf/cm$^2$ (i.e., 0.050 g/cc). This TBAM layer is preferably used as the absorbent core 50 in the embodiment shown in Figs. 1 and 2.

EXAMPLE 2

One layer of TBAM is produced from 65% of Pulp Fibers, 18% of Bico Fibers, and 17% of particulate AGM which exhibit 7.3 g/g of absorption with Test Fluid mentioned using Air-lay System and subsequent thermal treatment at 153 °C. This material has basis weight of about 100 g/m2 and thickness of about 2.0 mm at 20 gf/cm$^2$ (i.e. 0.050 g/cc). This TBAM layer is preferably used as the absorbent core 50 in the embodiment shown in Figs. 1 and 2.

EXAMPLE 3

One layer of TBAM is produced from 56% of Pulp Fibers, 18% of Bico Fibers, and 26% of particulate AGM which exhibit 7.3 g/g of absorption with Test Fluid mentioned using Air-lay System and subsequent thermal treatment at 150 °C. This material has basis weight of about 100 g/m2 and thickness of about 1.2 mm at 20 gf/cm$^2$ (i.e. 0.083 g/cc). This TBAM layer is preferably used as the absorbent core 50 in the embodiment shown in Figs. 1 and 2.

EXAMPLE 4

One layer of TBAM (A) is produced from 56% of Pulp Fibers, 18% of Bico Fibers, and 26% of particulate AGM which exhibit 7.3 g/g of absorption with Test Fluid mentioned using Air-lay System and subsequent thermal treatment at 156 °C. This TBAM (A) has basis weight of about 80 g/m2 and thickness of about 1.2 mm at 20 gf/cm$^2$ (i.e. 0.067 g/cc). Another layer of TBAM (B) is produced from 82% of Pulp Fibers, and 18% of Bico Fibers, using Air-lay System and subsequent thermal treatment at 138 °C. This TBAM has basis weight of about 200 g/m2 and thickness of about 4.0 mm at 20 gf/cm$^2$ (i.e. 0.050 g/cc). These TBAM layers (A) and (B) are preferably used as the lower and upper layers 59 and 58, respectively, in the embodiment shown in Figs. 3 and 4.

EXAMPLE 5

One layer of TBAM (C) is produced from 56% of Pulp Fibers, 18% of Bico Fibers, and 26% of particulate AGM which exhibit 7.3 g/g of absorption with Test Fluid mentioned using Air-lay System and subsequent thermal treatment at 156 °C. This TBAM has basis weight of about 80 g/m2 and thickness of about 1.2 mm at 20 gf/cm$^2$ (i.e. 0.067 g/cc). Another layer of TBAM (D) is produced from 65% of Pulp Fibers, 18% of Bico Fibers, and 17% of particulate AGM which exhibit 7.3 g/g of absorption with Test Fluid mentioned using Air-lay System and subsequent thermal treatment at 143 °C. This TBAM has basis weight of about 150 g/m2 and thickness of about 3.0 mm at 20 gf/cm$^2$ (i.e. 0.050 g/cc). These TBAM layers (C) and (D) are preferably used as the lower and upper layers 59 and 58, respectively, in the embodiment shown in Figs. 3 and 4.

EXAMPLE 6

One layer of TBAM (E) is produced from 56% of Pulp Fibers, 18% of Bico Fibers, and 26% of particulate AGM which exhibit 7.3 g/g of absorption with Test Fluid mentioned using Air-lay System and subsequent thermal treatment at 156 °C. This TBAM has basis weight of about 80 g/m2 and thickness of about 1.2mm at 20 gf/cm$^2$ (i.e. 0.067 g/cc). Another layer of TBAM (F) is produced from 65% of Pulp Fibers, 18% of Bico Fibers, and 17% of particulate AGM which exhibit 7.3 g/g of absorption with Test Fluid mentioned using Air-lay System and subsequent thermal treatment at 143 °C. This TBAM has basis weight of about 200 g/m2 and thickness of about 4.0 mm at 20 gf/cm$^2$ (i.e. 0.050 g/cc). These TBAM layers (E) and (F) are preferably used as the lower and upper layers 59 and 58, respectively, in the embodiment shown in Figs. 3 and 4.

**Claims**

1. An absorbent article, comprising:

   a topsheet;
   a backsheet; and
   an airlaid mixture material disposed between the topsheet and the backsheet, the airlaid mixture material including a) cellulosic fibers, and b) thermoplastic fibers having a surface treated with a surfactant including a phosphate ester, a sulfate ester, or a derivative thereof;
   the cellulosic fibers and the thermoplastic fibers being bonded together to form a thermally bonded airlaid matrix.

2. The absorbent article of the claim 1, wherein the phosphate ester or its derivative is selected from the group consisting of alkyl phosphate esters, alkyl phenol phosphate esters, and their derivatives.

3. The absorbent article of the claim 2, wherein the phosphate ester or its derivative includes a linear alkyl phosphate ester having a hydrocarbon chain length of from $C_6$ to $C_{22}$ in the form of an alkali salt.

4. The absorbent article of the claim 1, wherein the sulfate ester or its derivative is selected from the group consisting of alkyl sulfate esters, alkyl phenol sulfate esters, and their derivatives.

5. The absorbent article of the claim 4. wherein the sulfate ester or its derivative includes a linear alkyl sulfate ester having a hydrocarbon chain length of from $C_6$ to $C_{22}$ in the form of an alkali salt.

6. The absorbent article of the claim 1, wherein the thermally bonded airlaid matrix has dry density of from about 0.04 to about 0.11 g/cc under pressure of 20 gf/cm$^2$.

7. The absorbent article of the claim 1, wherein the thermoplastic fibers include a modified polyolefin to form a hydrogen bond or a covalent bond between the thermoplastic fibers and the cellulosic fibers.

8. The absorbent article of the claim 1, wherein the thermoplastic fibers are bicomponent fibers.

9. The absorbent article of the claim 1, wherein the airlaid material further includes an absorbent gelling material.

10. The absorbent article of the claim 9, wherein the absorbent gelling material is a hemophilic AGM.

11. The absorbent article of the claim 1, wherein the thermoplastic fibers includes from about 10% to about 30% of the thermoplastic fibers.

12. The absorbent article of the claim 9, wherein the absorbent gelling material includes from about 5% to about 50% of the absorbent gelling material.

13. The absorbent article of the claim 1. wherein the thermally bonded airlaid matrix includes first and second layers of thermally bonded airlaid matrix which are joined together, and the first layer is disposed closer to the topsheet than the second layer.

14. The absorbent article of the claim 13, wherein the first layer has a smaller area and greater thickness than the second layer.

15. The absorbent article of the claim 14, wherein the fist layer has an edge, and the absorbent article has an embossed channel which is formed on the second layer along the edge of the first layer.

**Patentansprüche**

1. Absorptionsartikel, umfassend:

   eine obere Lage;
   eine untere Lage; und
   ein luftgelegtes Mischmaterial, das zwischen der oberen Lage und der unteren Lage angeordnet ist, wobei das luftgelegte Mischmaterial a) Cellulosefasern und b) thermoplastische Fasern mit einer Oberfläche, die mit einem Tensid, einschließlich eines Phosphatesters, eines Sulfatesters oder eines Derivats davon, behandelt ist, einschließt;

   wobei die Cellulosefasern und die thermoplastischen Fasern zur Bildung einer thermisch gebundenen luftgelegten Matrix aneinander gebunden sind.

2. Absorptionsartikel nach Anspruch 1, wobei der Phosphatester oder dessen Derivat ausgewählt ist aus der Gruppe, bestehend aus Alkylphosphatestern, Alkylphenolphosphatestern und deren Derivaten.

3. Absorptionsartikel nach Anspruch 2, wobei der Phosphatester oder dessen Derivat einen linearen Alkylphosphatester mit einer Kohlenwasserstoffkettenlänge von $C_6$ bis $C_{22}$ in Form eines Alkalisalzes einschließt.

4. Absorptionsartikel nach Anspruch 1, wobei der Sulfatester oder dessen Derivat ausgewählt ist aus der Gruppe, bestehend aus Alkylsulfatestern, Alkylphenolsulfatestern und deren Derivaten.

5. Absorptionsartikel nach Anspruch 4, wobei der Sulfatester oder dessen Derivat einen linearen Alkylsulfatester mit einer Kohlenwasserstoffkettenlänge von $C_6$ bis $C_{22}$ in Form eines Alkalisalzes einschließt.

6. Absorptionsartikel nach Anspruch 1, wobei die thermisch gebundene luftgelegte Matrix eine Trockendichte von etwa 0,04 bis etwa 0,11 g/cm$^3$ unter einem Druck von 20 p/cm$^2$ aufweist.

7. Absorptionsartikel nach Anspruch 1, wobei die thermoplastischen Fasern ein modifiziertes Polyolefin einschließen, um eine Wasserstoffbindung oder eine kovalente Bindung zwischen den thermoplastischen Fasern und den Cellulosefasern zu bilden.

8. Absorptionsartikel nach Anspruch 1, wobei die thermoplastischen Fasern Bikomponentenfasern sind.

9. Absorptionsartikel nach Anspruch 1, wobei das luftgelegte Material ferner ein Absorptionsgeliermaterial einschließt.

10. Absorptionsartikel nach Anspruch 9, wobei das Absorptionsgeliermaterial ein hämophiles Absorptionsgeliermaterial ist.

11. Absorptionsartikel nach Anspruch 1, wobei die thermoplastischen Fasern von etwa 10 % bis etwa 30 % an thermoplastischen Fasern einschließen.

12. Absorptionsartikel nach Anspruch 9, wobei das Absorptionsgeliermaterial von etwa 5 % bis etwa 50 % an Absorptionsgeliermaterial einschließt.

13. Absorptionsartikel nach Anspruch 1, wobei die thermisch gebundene luftgelegte Matrix eine erste und eine zweite Schicht von thermisch gebundener luftgelegter Matrix einschließt, die miteinander verbunden sind, und die erste Schicht näher an der oberen Lage angeordnet ist als die zweite Schicht.

**14.** Absorptionsartikel nach Anspruch 13, wobei die erste Schicht eine kleinere Fläche und eine größere Dicke aufweist als die zweite Schicht.

**15.** Absorptionsartikel nach Anspruch 14, wobei die erste Schicht einen Rand aufweist und der Absorptionsartikel einen mit Prägungen versehenen Kanal aufweist, der auf der zweiten Schicht entlang des Randes der ersten Schicht gebildet wird.

**Revendications**

**1.** Article absorbant, comprenant:

une feuille de dessus;
une feuille de fond; et
un matériau de mélange appliqué par jet d'air disposé entre la feuille de dessus et la feuille de fond, le matériau de mélange appliqué par jet d'air incluant a) des fibres cellulosiques, et b) des fibres thermoplastiques ayant une surface traitée avec un agent tensioactif incluant un ester de phosphate, un ester de sulfate, ou un de ses dérivés;
les fibres cellulosiques et les fibres thermoplastiques étant liées ensemble de façon à former une matrice appliquée par jet d'air thermiquement liée.

**2.** Article absorbant selon la revendication 1, dans lequel l'ester de phosphate ou son dérivé est choisi dans le groupe constitué des esters de phosphate d'alkyle, des esters de phosphate d'alkylphénol, et leurs dérivés.

**3.** Article absorbant selon la revendication 2, dans lequel l'ester de phosphate ou son dérivé inclut un ester de phosphate d'alkyle linéaire ayant une longueur de chaîne hydrocarbonée de $C_6$ à $C_{22}$ sous la forme d'un sel alcalin.

**4.** Article absorbant selon la revendication 1, dans lequel l'ester de sulfate ou son dérivé est choisi dans le groupe constitué des esters de sulfate d'alkyle, des esters de sulfate d'alkylphénol, et leurs dérivés.

**5.** Article absorbant selon la revendication 4, dans lequel l'ester de sulfate ou son dérivé inclut un ester de sulfate d'alkyle linéaire ayant une longueur de chaîne hydrocarbonée de $C_6$ à $C_{22}$ sous la forme d'un sel alcalin.

**6.** Article absorbant selon la revendication 1, dans lequel la matrice appliquée par jet d'air thermiquement liée a une masse volumique sèche d'environ 0,04 à environ 0,11 g/c$^3$ sous une pression de 20 gf/cm$^2$.

**7.** Article absorbant selon la revendication 1, dans lequel les fibres thermoplastiques incluent une polyoléfine modifiée de façon à former une liaison hydrogène ou une liaison covalente entre les fibres thermoplastiques et les fibres cellulosiques.

**8.** Article absorbant selon la revendication 1, dans lequel les fibres thermoplastiques sont des fibres à deux composants.

**9.** Article absorbant selon la revendication 1, dans lequel le matériau appliqué par jet d'air inclut en outre un matériau absorbant et gélifiant.

**10.** Article absorbant selon la revendication 9, dans lequel le matériau absorbant et gélifiant est un MAG hémophile.

**11.** Article absorbant selon la revendication 1, dans lequel les fibres thermoplastiques incluent d'environ 10 % à environ 30 % des fibres thermoplastiques.

**12.** Article absorbant selon la revendication 9, dans lequel le matériau absorbant et gélifiant inclut d'environ 5 % à environ 50 % du matériau absorbant et gélifiant.

**13.** Article absorbant selon la revendication 1, dans lequel la matrice appliquée par jet d'air thermiquement liée inclut des première et deuxième couches de matrice appliquée par jet d'air thermiquement liée qui sont attachées ensemble, et la première couche est disposée plus près de la feuille de dessus que la deuxième couche.

**14.** Article absorbant selon la revendication 13, dans lequel la première couche a une surface inférieure et une épaisseur supérieure à la deuxième couche.

15. Article absorbant selon la revendication 14, dans lequel la première couche a un bord, et l'article absorbant a un canal gaufré qui est formé sur la deuxième couche le long du bord de la première couche.

**Fig. 1**

**Fig. 2**

**Fig. 3**

**Fig. 4**

Fig. 5